(19) Europäisches Patentamt
European Patent Office
Office européen des brevets

(11) Veröffentlichungsnummer: **0 009 762**
**B1**

# (12) EUROPÄISCHE PATENTSCHRIFT

(45) Veröffentlichungstag der Patentschrift: **05.08.81**

(51) Int. Cl.³: **C 07 C 127/22**, C 07 C 157/12, A 01 N 47/34

(21) Anmeldenummer: **79103647.8**

(22) Anmeldetag: **26.09.79**

(54) **Substituierte N-Benzoyl-N'-tert.-alkoxycarbonylphenyl-(thio)harnstoffe, Verfahren zu ihrer Herstellung und ihre Verwendung als Insektizide.**

(30) Priorität: **07.10.78 DE 2843851**

(43) Veröffentlichungstag der Anmeldung: **16.04.80 Patentblatt 80/8**

(45) Bekanntmachung des Hinweises auf die Patenterteilung: **05.08.81 Patentblatt 81/31**

(84) Benannte Vertragsstaaten: **AT BE CH DE FR GB IT NL SE**

(56) Entgegenhaltungen: **CHEMICAL ABSTRACTS, Vol. 64, Nr. 3, 31. Januar 1966, Sp. 3432d, Columbus, Ohio, USA**

(73) Patentinhaber: **BAYER AG, Zentralbereich Patente, Marken und Lizenzen, D-5090 Leverkusen 1, Bayerwerk (DE)**

(72) Erfinder: **Sirrenberg, Wilhelm, Dr., Wuppertaler Strasse 21, D-4322 Sprockhövel (DE)**
Erfinder: **Hammann, Ingeborg, Dr., Belfortstrasse 9, D-5000 Köln 1 (DE)**
Erfinder: **Stendel, Wilhelm, Dr., In den Birken 55, D-5600 Wuppertal 1 (DE)**
Erfinder: **Klauke, Erich, Dr., Eichendorffweg 8, D-5068 Odenthal (DE)**

**EP 0 009 762 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Substituierte N-Benzoyl-N'-tert.-alkoxycarbonylphenyl-(thio)harnstoffe, Verfahren zu ihrer Herstellung und ihre Verwendung als Insektizide

Die Erfindung betrifft neue substituierte N-Benzoyl-N'-tert.-alkoxycarbonylphenyl-(thio)harnstoffe, mehrere Verfahren zu ihrer Herstellung und ihre Vewendung als Insektizide.

Es ist bekannt, dass bestimmte Benzoylharnstoffe und Benzoylthioharnstoffe, wie z.B. N-(2,6-Dichlorbenzoyl)-N'-(3,4-dichlorphenyl)-harnstoff und N-(2,6-Dichlorbenzoyl)-N'-(3,4-dichlorphenyl)-thioharnstoff, insektizide Eigenschaften aufweisen (vergleiche US-PS 3 933 908).

Die insektizide Wirkung dieser Verbindungen ist jedoch, insbesondere bei niedrigen Wirkstoffkonzentrationen und Aufwandmengen, nicht immer zufriedenstellend.

Es wurden nun neue N-Benzoyl-N'-tert.-alkoxycarbonylphenyl-(thio)harnstoffe der Formel I

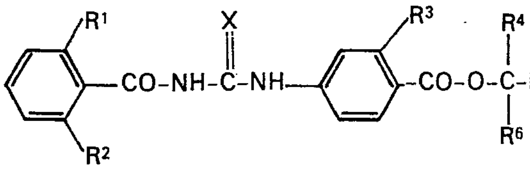

(I)

gefunden, in welcher $R^1$ für Halogen oder Alkyl steht,
$R^2$ und
$R^3$ unabhängig voneinander für Wasserstoff oder Halogen stehen,
$R^4$ für Alkyl steht,
$R^5$ und
$R^6$ entweder einzeln für Alkyl oder zusammen für α,ω-Alkandiyl stehen und
X für Sauerstoff oder Schwefel steht.

Man erhält die neuen N-Benzoyl-N'-tert.-alkoxycarbonylphenyl-(thio)harnstoffe der Formel (I), wenn man

a) substituierte Benzoyl-iso(thio)cyanate der Formel II

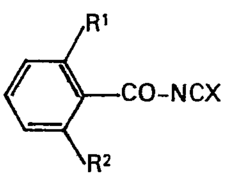

(II)

in welcher $R^1$, $R^2$ und X die oben angegebene Bedeutung haben, mit Amino-benzoesäureestern der Formel III

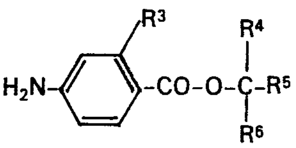

(III)

in welcher $R^3$, $R^4$, $R^5$ und $R^6$ die oben angegebene Bedeutung haben, gegebenenfalls unter Verwendung eines Verdünnungsmittels umsetzt oder

b) substituierte Benzoesäureamide der Formel IV

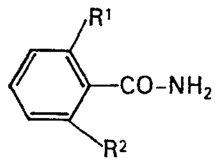

(IV)

in welcher $R^1$ und $R^2$ die oben angegebene Bedeutung haben, mit tert.-Alkoxycarbonylphenyl-iso(thio)cyanaten der Formel V

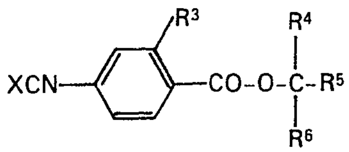

(V)

in welcher $R^3$, $R^4$, $R^5$, $R^6$ und X die oben angegebene Bedeutung haben, gegebenenfalls unter Verwendung eines Verdünnungsmittels umsetzt.

Überraschenderweise zeigen die erfindungsgemässen Verbindungen der Formel (I) in vergleichenden Versuchen mit N-(2,6-Dichlorbenzoyl)-N'-(3,4-dichlorphenyl)-harnstoff und dem entsprechenden Thioharnstoff eine wesentlich stärkere insektizide Wirkung als die aus dem Stand der Technik bekannten Wirkstoffe analoger Konstitution und gleicher Wirkung.

Gegenstand der Erfindung sind vorzugsweise N-Benzoyl-N'-tert.-alkoxycarbonylphenyl-(thio)harnstoffe der Formel (I), in welcher
$R^1$ für Fluor, Chlor, Brom, Jod oder Methyl steht,
$R^2$ für Wasserstoff, Fluor, Chlor, Brom oder Jod steht,
$R^3$ für Wasserstoff, Chlor oder Brom steht,
$R^4$ für Methyl oder Äthyl steht,
$R^5$ und $R^6$ entweder einzeln für geradkettiges oder verzweigtes Alkyl mit 1 bis 4 Kohlenstoffatomen, insbesondere für Methyl oder Äthyl, oder zusammen für geradkettiges α,ω-Alkandiyl mit 5 bis 7 Kohlenstoffatomen stehen und
X für Sauerstoff oder Schwefel steht.

Verwendet man beispielsweise 2-Fluorbenzoyl-isothiocyanat und 4-Amino-benzoesäure-tert.-butylester als Ausgangsstoffe bei Verfahren (a) bzw. 2,6-Dibrombenzamid und 4-tert.-Butoxycarbonylphenyl-isocyanat als Ausgangsstoffe bei Verfahren (b), so können die entsprechenden Reaktionen durch folgende Formelschemata skizziert werden:

(a)

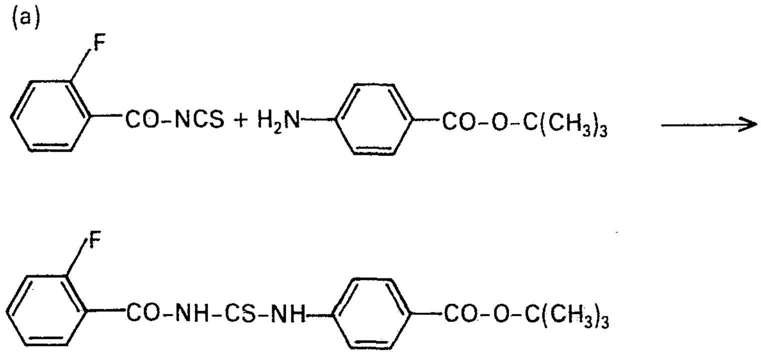

(b)

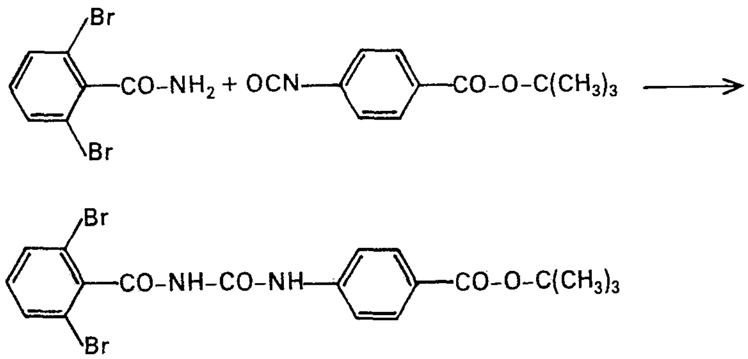

Die zu verwendenden Ausgangsverbindungen sind durch die Formeln (II), (III), (IV) und (V) definiert. Vorzugsweise stehen darin $R^1$ bis $R^6$ für diejenigen Reste, welche bei der Definition der Reste $R^1$ bis $R^6$ in Formel (I) als bevorzugt genannt wurden.

Die als Ausgangsverbindungen zu verwendenden Benzoesäureamide (IV) und die entsprechenden Benzoyl-iso(thio)cyanate (II) sind bekannt oder können analog bekannten Verfahren hergestellt werden (vergleiche z.B. J.Org. Chem. 30 (1965), 4306-4307 und DE-AS 1 215 144).

Als Beispiele seien genannt:
2-Fluor-, 2-Chlor-, 2-Brom-, 2-Jod-, 2-Methyl-, 2,6-Difluor-, 2,6-Dichlor-, 2,6-Dibrom- und 2-Chlor-6-fluorbenzoesäureamid;
2-Fluor-, 2-Chlor-, 2-Brom-, 2-Jod-, 2-Methyl-, 2,6-Difluor-, 2,6-Dichlor-, 2,6-Dibrom- und 2-Chlor-6-fluorbenzoylisocyanat sowie
2-Fluor-, 2-Chlor-, 2-Brom-, 2-Jod-, 2-Methyl-, 2,6-Difluor-, 2,6-Dichlor-, 2,6-Dibrom- und 2-Chlor-6-fluorbenzoylisothiocyanat.

Die als weitere Ausgangsstoffe zu verwendenden Aminobenzoesäureester (III) sind bekannt oder können analog bekannten Verfahren hergestellt werden (vergleiche z.B. J. Am. Chem. Soc. 66 (1944), 1781; Arzneimittel-Forschung 1968, 791-798; Chem. Abstr. 69 (1968), 65 894p).

Als Beispiele seien genannt:
4-Amino-benzoesäure-tert.-butylester und -tert.-pentylester sowie 4-Amino-2-chloro-benzoesäure-tert.-butylester und -tert.-pentylester.

Aus den Aminobenzoesäureestern der Formel (III) können die entsprechenden 4-tert.-Alkoxycarbonylphenylisocyanate und -isothiocyanate der Formel (V) nach üblichen Methoden, z.B. durch Umsetzung mit Phosgen bzw. mit Thiophosgen, hergestellt werden (vergleiche J. Chem. Soc. 1934, 178 und Houben-Weyl, Methoden der organischen Chemie, 4. Auflage, Band 8, S. 120, Georg Thieme Verlag, Stuttgart 1952).

Als Beispiele seien genannt:
4-tert.-Butoxycarbonylphenyl-isocyanat und -isothiocyanat, 4-tert.-Pentoxycarbonylphenylisocyanat und -isothiocyanat, 3-Chloro-4-tert.-butoxycarbonylphenylisocyanat und -isothiocyanat sowie 3-Chloro-4-tert.-pentoxycarbonylphenyl-isocyanat und -isothiocyanat.

Die Verfahren zur Herstellung der erfindungsgemässen N-Benzoyl-N′-tert.-alkoxycarbonylphenyl-(thio)harnstoffe werden bevorzugt unter Verwendung geeigneter Lösungs- oder Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- udn Dibutyläther, Tetrahydrofuran und Dioxan, Ketone wie Aceton, Methyläthyl-, Methylisopropyl- und Methylisobutylketon sowie Nitrile wie Acetonitril und Propionitril.

Die Reaktionstemperaturen können innerhalb eines grösseren Bereichs variiert werden. Im allgemeinen arbeitet man zwischen 0 und 150 °C, vorzugsweise bei 10 bis 100 °C.

Die erfindungsgemässen Verfahren werden im allgemeinen bei Normaldruck durchgeführt.

Zur Durchführung der erfindungsgemässen Verfahren werden die Ausgangsstoffe gewöhnlich in äquimolaren Mengen eingesetzt. Ein Überschuss der einen oder anderen Reaktionskomponente bringt keine wesentlichen Vorteile. Die Umsetzung wird im allgemeinen in einem geeigneten Verdünnungsmittel durchgeführt und das Reaktionsgemisch wird mehrere Stunden bei der erforderlichen Temperatur gerührt. Danach lässt man das Reakionsgemisch abkühlen und saugt vom auskristallisierten Produkt ab. Zur Charakterisierung dient der Schmelzpunkt.

Die erfindungsgemässen N-Benzoyl-N'-tert.-alkoxycarbonylphenyl-(thio)harnstoffe zeichnen sich durch eine hervorragende insektizide Wirksamkeit aus. Sie können gegen pflanzenschädigende Insekten im Pflanzenschutz, gegen Hygiene- und Vorratsschädlinge wie auch gegen Ektoparasiten im veterinär-medizinischen Bereich als Schädlingsbekämpfungsmittel eingesetzt werden.

Die Wirkstoffe eignen sich bei gutzer Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Crytpomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecaniumcorni, Saissetia oleae, Laodelphax striatellus, Nilaparvata lugens, Aonidiela aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padela, Plutella maculipennis, Malocosoma neustria, Euproctis chrysorrhoea, Lymantria spp. Bucculatrix thurberiela, Phyllocnistis citrella, Agrotis spp., Euxoa spp., Feltia spp. Earias insulana, Heliothis spp., Laphygma exigua, Mamestra brassicae, Panolis flammea, Prodenialitura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthosecelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnusschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von Ekto- und Endoparasiten auf dem veterinärmedizinischen Gebiet.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuderns sowie durch parenterale Anwendung in Form beispielsweise der Injektion.

Beispiel A

Plutella-Test

Lösungsmittel: 3 Gewichtsteile Dimethylformamid

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen z.B. die folgenden Verbindungen der Herstellungsbeispiele überlegene Wirksamkeit gegenüber dem Stand der Technik: 18, 17, 20, 22, 2, 6, 1, 8, 5, 9.

Beispiel B

Test mit Lucilia cuprina res.-Larven

Lösungsmittel: 35 Gewichtsteile Äthylenglykolmonomethyläther

Emulgator: 35 Gewichtsteile Nonylphenolpolyglykoläther

Zur Herstellung einer zweckmässigen Wirkstoffzubereitung vermischt man drei Gewichtsteile Wirkstoff mit sieben Gewichtsteilen des oben angegebenen Lösunsmittel-Gemisches und verdünnt das so erhaltene Konzentrat mit Wasser auf die jeweils gewüschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele 1, 9, 5 und 21 eine besonders günstige Wirkung.

Herstellungsbeispiele

Beispiel 1

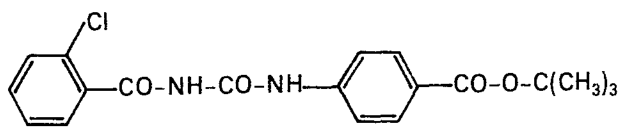

Zu einer Lösung von 5,79 g (0,03 Mol) 4-Aminobenzoesäure-tert.-butylester in 60 ml Toluol fügt man bei 60 °C 5,5 g 2-Chlorbenzoylisocyanat in 20 ml Toluol. Der Ansatz wird eine Stunde bei 80 °C gerührt und anschliessend auf 20 °C abgekühlt. Das kristalline Produkt wird abgesaugt und getrocknet. Es schmilzt bei 171 °C. Seine Identifizierung erfolgte durch Elementaranalyse und NMR-spektroskopisch. Die Ausbeute beträgt 7,6 g (68% der Theorie) N-(2-Chloro-benzoyl)-N'-(4-tert.-butoxycarbonylphenyl)-harnstoff.

Beispiel 2

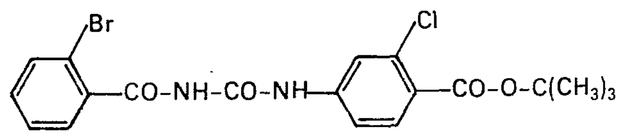

Eine Lösung von 4,55 g (0,02 Mol) 2-Chlor-4-aminobenzoesäure-tert.-butylester in 50 ml Toluol wird mit einer Lösung von 4,92 g 2-Brombenzoylisocyanat in 20 ml Toluol versetzt und 1 Stunde bei 80 °C gerührt. Beim Abkühlen auf Raumtemperatur kristallisiert das Produkt aus. Es wird abgesaugt und getrocknet. Die Substanz schmilzt bei 197 °C. Die Identifizierung erfolgte durch Elementaranalyse und NMR-spektroskopisch. Die Ausbeute beträgt 7,8 g (86% der Theorie) N-(2-Bromo-benzoyl)-N'-(3-chloro-4-tert.-butoxycarbonylphenyl)-harnstoff.

Analog einem der Beispiele 1 und/oder 2 wurden folgende Verbindungen der allgemeinen Formel I

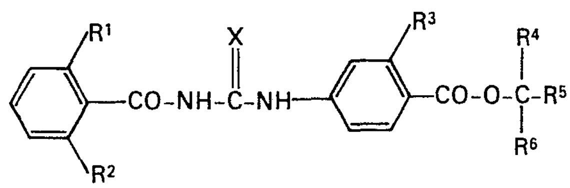

hergestellt:

| Beispiel Nr. | $R^1$ | $R^2$ | $R^3$ | $R^4$ | $R^5$ | $R^6$ | X | Schmelzpunkt (°C) | Ausbeute (% der Theorie) |
|---|---|---|---|---|---|---|---|---|---|
| 3 | $CH_3$ | H | H | $CH_3$ | $CH_3$ | $CH_3$ | 0 | 180 | 88 |
| 4 | F | H | H | $CH_3$ | $CH_3$ | $CH_3$ | 0 | 175 | 86,5 |
| 5 | Br | H | H | $CH_3$ | $CH_3$ | $CH_3$ | 0 | 200 | 74,5 |
| 6 | J | H | H | $CH_3$ | $CH_3$ | $CH_3$ | 0 | 203 | 82 |
| 7 | Cl | F | H | $CH_3$ | $CH_3$ | $CH_3$ | 0 | 181 | 97,5 |
| 8 | Cl | Cl | H | $CH_3$ | $CH_3$ | $CH_3$ | 0 | 200–201 | 63,5 |
| 9 | F | F | H | $CH_3$ | $CH_3$ | $CH_3$ | 0 | 193 | 81 |
| 10 | $CH_3$ | H | H | $CH_3$ | $CH_3$ | $CH_3$ | S | 144 | 71,5 |
| 11 | Cl | H | H | $CH_3$ | $CH_3$ | $CH_3$ | S | 144 | 60 |
| 12 | Br | H | H | $CH_3$ | $CH_3$ | $CH_3$ | S | 156 | 77 |
| 13 | Cl | F | H | $CH_3$ | $CH_3$ | $CH_3$ | S | 193 | 99 |
| 14 | Cl | Cl | H | $CH_3$ | $CH_3$ | $CH_3$ | S | 219 | 86 |
| 15 | F | F | H | $CH_3$ | $CH_3$ | $CH_3$ | S | 161 | 93 |
| 16 | $CH_3$ | H | Cl | $CH_3$ | $CH_3$ | $CH_3$ | 0 | 153 | 78,5 |
| 17 | F | H | Cl | $CH_3$ | $CH_3$ | $CH_3$ | 0 | 165 | 86,5 |
| 18 | Cl | H | Cl | $CH_3$ | $CH_3$ | $CH_3$ | 0 | 180 | 90,5 |
| 19 | J | H | Cl | $CH_3$ | $CH_3$ | $CH_3$ | 0 | 210 | 87,5 |

| Beispiel Nr. | $R^1$ | $R^2$ | $R^3$ | $R^4$ | $R^5$ | $R^6$ | X | Schmelzpunkt (°C) | Ausbeute (% der Theorie) |
|---|---|---|---|---|---|---|---|---|---|
| 20 | Cl | F | Cl | $CH_3$ | $CH_3$ | $CH_3$ | 0 | 207(Z) | 90,5 |
| 21 | Cl | Cl | Cl | $CH_3$ | $CH_3$ | $CH_3$ | 0 | 162 | 72,5 |
| 22 | F | F | Cl | $CH_3$ | $CH_3$ | $CH_3$ | 0 | 200 | 92,5 |
| 23 | $CH_3$ | H | Cl | $CH_3$ | $CH_3$ | $CH_3$ | S | 130 | 88 |
| 24 | Cl | H | Cl | $CH_3$ | $CH_3$ | $CH_3$ | S | 150 | 86 |
| 25 | Br | H | Cl | $CH_3$ | $CH_3$ | $CH_3$ | S | 137 | 66 |
| 26 | Cl | F | Cl | $CH_3$ | $CH_3$ | $CH_3$ | S | 173 | 92,5 |
| 27 | Cl | Cl | Cl | $CH_3$ | $CH_3$ | $CH_3$ | S | 186 | 87 |
| 28 | F | F | Cl | $CH_3$ | $CH_3$ | $CH_3$ | S | 145 | 89 |
| 29 | Cl | H | H | $CH_3$ | $CH_3$ | $C_2H_5$ | 0 | 140 | 87,5 |
| 30 | Br | H | H | $CH_3$ | $CH_3$ | $C_2H_5$ | 0 | 163 | 76 |
| 31 | Cl | F | H | $CH_3$ | $CH_3$ | $C_2H_5$ | 0 | 178 | 77,5 |
| 32 | $CH_3$ | H | H | $CH_3$ | $CH_3$ | $C_2H_5$ | S | 170 | 84,5 |
| 33 | Cl | H | H | $CH_3$ | $CH_3$ | $C_2H_5$ | S | 92 | 85,5 |
| 34 | Br | H | H | $CH_3$ | $CH_3$ | $C_2H_5$ | S | 103 | 92,5 |
| 35 | Cl | F | H | $CH_3$ | $CH_3$ | $C_2H_5$ | S | 174 | 92 |
| 36 | Cl | Cl | H | $CH_3$ | $CH_3$ | $C_2H_5$ | S | 190 | 78,5 |

Beispiel 37

Herstellung nach Verfahren b

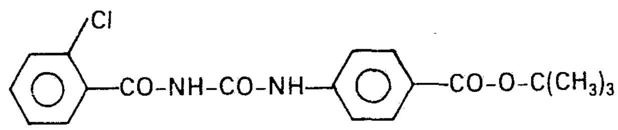

Zu 3,1 g 2-Chlorbenzamid (0,02 Mol) in 100 ml Toluol fügt man 4,38 g 4-tert.-Butoxycarbonyl-phenyl-isocyanat (Kpz. 108°C; $n_D^{25}$: 1,5242) in 20 ml Toluol und rührt den Ansatz 16 Stunden bei 10°C. Anschliessend saugt man ausgefallene Festsubstanz warm ab und isoliert aus dem Filtrat 3,5 g (46,5% der Theorie) des gewünschten Produktes mit einem Schmelzpunkt von 171 °C.

**Patentansprüche**

1. N-Benzoyl-N'-tert.-alkoxycarbonylphenyl-(thio)harnstoffe der Formel (I)

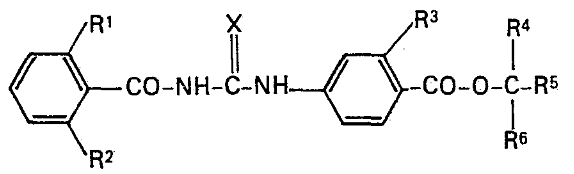

(I)

in welcher $R^1$ für Halogen oder Alkyl steht,
$R^2$ und
$R^3$ unabhängig von einander für Wasserstoff oder Halogen stehen,
$R^4$ für Alkyl steht,
$R^5$ und
$R^6$ entweder einzeln für Alkyl oder zusammen für α,ω-Alkandiyl stehen und
X für Sauerstoff oder Schwefel steht.

2. Verfahren zur Herstellung der N-Benzoyl-N'-tert.-alkoxycarbonyl-phenyl-(thio)harnstoffe der Formel I

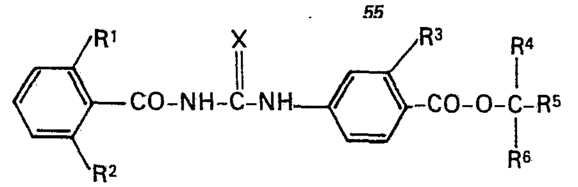

(I)

in welcher $R^1$ für Halogen oder Alkyl steht,
$R^2$ und
$R^3$ unabhängig von einander für Wasserstoff oder Halogen stehen,
$R^4$ für Alkyl steht,
$R^5$ und

$R^6$ entweder einzeln für Alkyl oder zusammen für α,ω-Alkandiyl stehen und
X für Sauerstoff oder Schwefel steht,
dadurch gekennzeichnet, dass man
a) substituierte Benzoyl-iso(thio)cyanate der Formel II

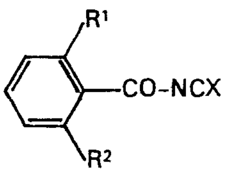

(II)

in welcher $R^1$, $R^2$ und X die oben angegebene Bedeutung haben, mit Amino-benzoesäureestern der Formel III

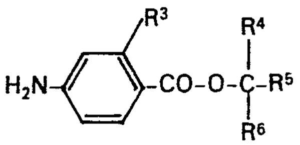

(III)

in welcher $R^3$, $R^4$, $R^5$ und $R^6$ die oben angegebene Bedeutung haben, gegebenenfalls unter Verwendung eines Verdünnungsmittels umsetzt oder
b) substituierte Benzoesäureamide der Formel IV

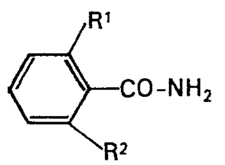

(IV)

in welcher $R^1$ und $R^2$ die oben angegebene Bedeutung haben, mit tert.-Alkoxycarbonylphenyl-iso(thio)cyanaten der Formel V

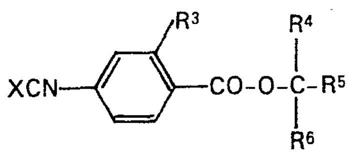

(V)

in welcher $R^3$, $R^4$, $R^5$, $R^6$ und X die oben angegebene Bedeutung haben, gegebenenfalls unter Verwendung eines Verdünnungsmittels umsetzt.

3. Insektizide Mittel, gekennzeichnet durch einen Gehalt an mindestens einem N-Benzoyl-N'-tert.-alkoxycarbonylphenyl-(thio)harnstoff der Formel (I).

4. Verwendung von N-Benzoyl-N'-tert.-alkoxycarbonylphenyl-(thio)harnstoffe der Formel (I) zur Bekämpfung von Insekten.

5. Verfahren zur Herstellung insektizider Mittel, dadurch gekennzeichnet, dass man N-Benzoyl-N'-tert.-alkoxycarbonylphenyl-(thio)harnstoffe der Formel (I) mit Streckmitteln und/oder oberflächenaktiven Mitteln vermischt.

**Claims**

1. N-Benzoyl-N'-tert.-alkoxycarbonylphenyl-(thio)ureas of the formula (I)

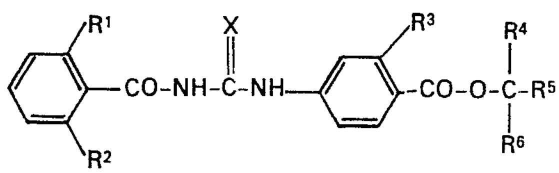

(I)

in which $R^1$ represents halogen or alkyl,
$R^2$ and $R^3$ independently of one another represent hydrogen or halogen,
$R^4$ represents alkyl,
$R^5$ and $R^6$ either individually represent alkyl or together represent α,ω-alkanediyl and
X represents oxygen or sulphur.

2. Process for the preparation of the N-benzoyl-N'-tert.-alkoxycarbonyl-phenyl-(thio)ureas of the formula (I),

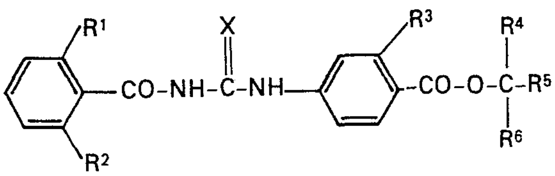

(I)

in which $R^1$ represents halogen or alkyl,
$R^2$ and $R^3$ independently of one another represent hydrogen or halogen,
$R^4$ represents alkyl,
$R^5$ and $R^6$ either individually represent alkyl or together represent α,ω-alkanediyl and
X represents oxygen or sulphur,
characterised in that
a) substituted benzoyl iso(thio)cyanates of the formula II

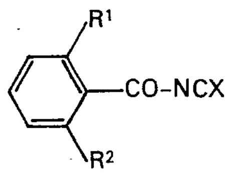

(II)

in which $R^1$, $R^2$ and X have the meaning indicated above, are reacted with amino-benzoic acid esters of the formula III

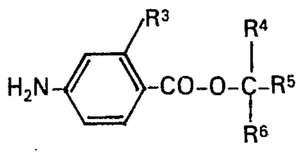

(III)

in which $R^3$, $R^4$, $R^5$ and $R^6$ have the meaning indicated above, if appropriate using a diluent, or

b) substituted benzoic acid amides of the formula IV

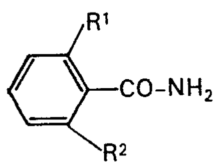

(IV)

in which $R^1$ and $R^2$ have the meaning indicated above, are reacted with tert.-alkoxycarbonylphenyl iso(thio)cyanates of the formula V

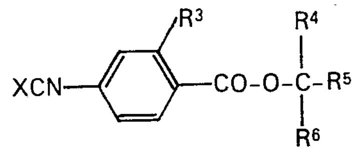

(V)

in which $R^3$, $R^4$, $R^5$, $R^6$ and X have the meaning indicated above, if appropriate using a diluent.

3. Insecticidal agents, characterised in that they contain at least one N-benzoyl-N'-tert.-alkoxycarbonylphenyl-(thio)urea of the formula (I).

4. Use of N-benzoyl-N'-tert.-alkoxycarbonylphenyl-(thio)ureas of the formula (I) for combating insects.

5. Process for the preparation of insecticidal agents, characterised in that N-benzoyl-N'-tert.-alkoxycarbonylpehnyl-(thio)ureas of the formula (I) are mixed with extenders and/or surface-active agents.

**Revendications**

1. N-benzoyl-N'-tert-alcoxycarbonylphényl-(thio)urées de formule I

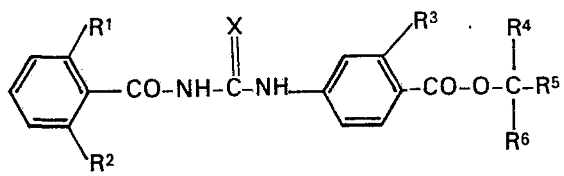

(I)

dans laquelle:
$R^1$ représente un halogène ou un groupe alkyle,
$R^2$ et $R^3$ représentent chacun, indépendamment l'un de l'autre, l'hydrogène ou un halogène,
$R^4$ représente un groupe alkyle,
$R^5$ et $R^6$ représentent chacun un groupe alkyle ou forment ensemble un groupe $\alpha,\omega$-alcane-diyle et
X représente l'oxygène ou le soufre.

2. Procédé de préparation des N-benzoyl-N'-tert-alcoxycarbonylphényl-(thio)urées de formule I

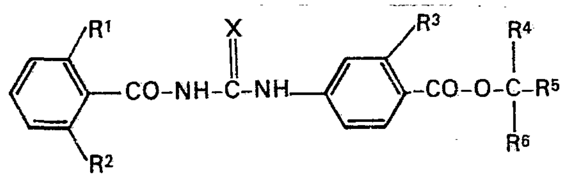

(I)

dans laquelle:
$R^1$ représente un halogène ou un groupe alkyle,
$R^2$ et $R^3$ représentent chacun, indépendamment l'un de l'autre, l'hydrogène ou un halogène,
$R^4$ représente un groupe alkyle,
$R^5$ et $R^6$ représentent chacun un groupe alkyle ou forment ensemble un groupe $\alpha,\omega$-alcane-diyle, et
X représente l'oxygène ou le soufre,
caractérisé en ce que:
a) on fait réagir des benzoyl-iso(thio)cyanates substitués de formule II

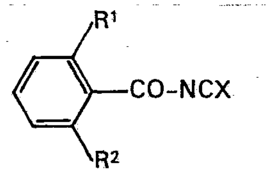

(II)

dans laquelle $R^1$, $R^2$ et X ont les significations indiquées ci-dessus, avec des esters aminobenzoïques de formule III

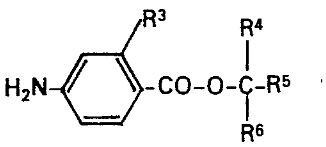

(III)

dans laquelle $R^3$, $R^4$, $R^5$ et $R^6$ ont les significations indiquées ci-dessus, le cas échéant, avec utilisation d'un diluant, ou bien

b) on fait réagir des benzamides substitués de formule IV

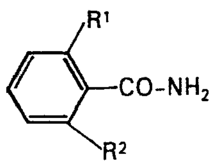

(IV)

dans laquelle $R^1$ et $R^2$ ont les significations indiquées ci-dessus, avec des tert-alcoxycarbonylphényliso(thio)cyanates de formule V

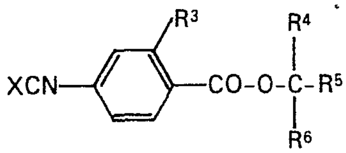

(V)

dans laquelle $R^3$, $R^4$, $R^5$, $R^6$ et X ont les significations indiquées ci-dessus, le cas échéant, en utilisant un diluant.

3. Produit insecticide, caractérisé en ce qu'il contient au moins une N-benzoyl-N'-tert-alcoxycarbonylphényl-(thio)urée de formule I.

4. Utilisation des N-benzoyl-N'-tert-alcoxycarbonylphényl-(thio)urées de formule I pour la lutte contre les insectes.

5. Procédé de préparation de produits insecticides, caractérisé en ce que l'on mélange des N-benzoyl-N'-tert-alcoxycarbonylphényl-(thio)urées de formule I avec des diluants et/ou des agents tensioactifs.